# EUROPÄISCHE PATENTSCHRIFT

(11) **EP 4 520 390 B1**
(45) Veröffentlichungstag und Bekanntmachung des Hinweises auf die Patenterteilung: **13.05.2026**
(21) Anmeldenummer: 24196988.0
(22) Anmeldetag: 28.08.2024
(51) Int. Cl.: A61N 1/05, A61M 25/00, A61M 25/02, A61M 25/06

(54) **SET ZUR NERVENSTIMULATION**
SET FOR NERVE STIMULATION
ENSEMBLE DE STIMULATION NERVEUSE

(30) Priorität: 05.09.2023 DE 102023123897
(43) Veröffentlichungstag der Anmeldung: 12.03.2025
(73) Patentinhaber: Pajunk GmbH Medizintechnologie, 78187 Geisingen (DE)
(72) Erfinder: PAJUNG-SCHELLING, Simone, 78187 Geisingen (DE); HAUGER, Martin, 78166 Donaueschingen (DE)
(74) Vertreter: Westphal, Mussgnug & Partner Patentanwälte mbB

(56) Entgegenhaltungen:
- WO-A1-97/15347
- DE-U1- 29 820 525
- US-A1- 2004 073 159
- US-A1- 2009 287 140

## Beschreibung

Die Erfindung betrifft ein Set zur Nervenstimulation.

Es ist bekannt, zur Schmerzlinderung oder zur Lösung von muskulären Verspannungen elektrische Strompulse, beispielsweise niedrigfrequenten Wechselstrom, mit niedriger Spannung anhand von auf der Haut platzierten Elektroden zu übertragen, um auf diese Weise transkutan die Nerven zu stimulieren. Dabei stellt die Haut jedoch einen vergleichsweise großen Übergangswiderstand dar. Zudem erfolgt die Stimulation in der Regel großflächig.

Es ist weiterhin bekannt, starre Kanülen, welche durch die Haut eingestochen werden, insbesondere auch durch eine Verweilkanüle geführt, mit einem Stromanschluss stimulierbar auszugestalten, um Rückschlüsse über die Positionierung der Spitze der Kanüle zu erhalten, insbesondere derart, ob die Spitze der Kanüle an einem Nerv anliegt.

Als Stand der Technik werden die WO 97/15347 A1, US 2009/287140 A1, DE 298 20 525 U1 und US 2004/073159 A1 genannt.

Es ist Aufgabe der Erfindung, eine verbesserte Möglichkeit zur Nervenstimulation anzugeben, welche insbesondere vielseitig einsetzbar ist und mit welcher vorzugsweise eine direktere Stimulation möglich ist.

Die Aufgabe der Erfindung wird gelöst durch ein Set zur Nervenstimulation mit den Merkmalen des Patentanspruchs 1.

Vorteilhafte Ausgestaltungen und Weiterbildungen der Erfindung sind in den abhängigen Ansprüchen angegeben.

Das erfindungsgemäße Set zur Nervenstimulation umfasst eine Vorrichtung zur Nervenstimulation mit einem distalen Ende, wobei die Vorrichtung ein elektrisch leitendes Stimulationselement und einen Kanal zur Zufuhr eines Fluids umfasst, und umfasst weiterhin eine Verweilkanüle mit einem Verweilkanülenrohr mit einem distalen Ende und einem proximalen Ende, wobei die Vorrichtung ein erstes Konnektorteil und die Verweilkanüle ein zu dem ersten Konnektorteil komplementäres zweites Konnektorteil aufweist, wobei durch Konnektieren des ersten Konnektorteils und des zweiten Konnektorteils die Vorrichtung in einer axialen Position festlegbar ist, in welcher das distale Ende der Vorrichtung über das distale Ende der Verweilkanüle hervorragt, wobei im konnektierten Zustand des ersten Konnektorteils und des zweiten Konnektorteils das erste Konnektorteil und das zweite Konnektorteil derart miteinander verbunden sind, dass eine umlaufende erste Dichtung gebildet ist.

Das erfindungsgemäße Set eignet sich sowohl zur Nervenstimulation als auch zur Zufuhr eines Fluids, beispielsweise einer Flüssigkeit wie eines Anästhetikums. Über die Verweilkanüle kann die Vorrichtung längerfristig im Körper positioniert werden und ermöglicht vielfältige Anwendungen. Das Verweilkanülenrohr kann aus einem Kunststoff, vorzugsweise einem Polyamid, gefertigt sein und vorzugsweise elektrisch isolierend ausgebildet sein. Insbesondere weist das Verweilkanülenrohr eine höhere Biegesteifigkeit auf als der in die Verweilkanüle eingeführte Teil der Vorrichtung zur Nervenstimulation, wodurch das Vorschieben der Vorrichtung durch das Verweilkanülenrohr vereinfacht wird. Andererseits ist eine flexible Ausgestaltung des Verweilkanülenrohrs wünschenswert, um auch bei langfristiger Anordnung der Verweilkanüle im Körper möglichst wenig störend auf den Patienten zu wirken.

Durch die umlaufende erste Dichtung wird der Raum zwischen der Vorrichtung zur Nervenstimulation und der Verweilkanüle abgedichtet, so dass die Möglichkeit besteht, in diesen Raum Fluid einzuführen. Dadurch kann sich der Vorteil ergeben, dass die in der Verweilkanüle angeordnete Vorrichtung zur Nervenstimulation über die gesamte Länge der Verweilkanüle verbessert mittels Ultraschall beobachtbar ist.

Vorzugsweise ist die erste Dichtung durch Anlage einer ersten Kontaktfläche des ersten Konnektorteils an einer zweiten Kontaktfläche des zweiten Konnektorteils gebildet, wodurch zusätzliche Komponenten wie beispielsweise ein Dichtungsring vermieden werden können.

Gemäß einer vorteilhaften Weiterbildung weist das erste Konnektorteil eine distale Fläche auf, auf welcher ein in axialer Richtung vorstehender umlaufender erster Vorsprung angeordnet ist, und weiterhin weist das zweite Konnektorteil eine proximale Fläche auf, auf welcher ein in axialer Richtung vorstehender umlaufender zweiter Vorsprung angeordnet ist, wobei die erste Kontaktfläche auf der Außenseite des ersten Vorsprungs und die zweite Kontaktfläche auf der Innenseite des zweiten Vorsprungs oder alternativ die erste Kontaktfläche auf der Innenseite des ersten Vorsprungs und die zweite Kontaktfläche auf der Außenseite des zweiten Vorsprungs angeordnet ist. Auf diese Weise kann sich über einen in axialer Richtung verlaufenden Abschnitt eine umlaufende Anlagefläche zwischen der ersten Kontaktfläche und der zweiten Kontaktfläche ergeben, welche eine zuverlässige Bildung der ersten Dichtung ermöglicht.

Vorzugsweise weist die erste Kontaktfläche einen in Richtung auf die zweite Kontaktfläche vorstehenden umlaufenden Wulst und/oder die zweite Kontaktfläche einen in Richtung auf die erste Kontaktfläche vorstehenden umlaufenden Wulst auf, welcher insbesondere bewirkt, dass der erste Vorsprung und der zweite Vorsprung unter Vorspannung gegeneinander anliegen, wodurch die Dichtwirkung verbessert werden kann.

Gemäß der Erfindung ist vorgesehen, dass das zweite Konnektorteil an einem distalen Ende das Verweilkanülenrohr abdichtend umschließt und sich in Richtung auf ein proximales Ende aufweitet, so dass angrenzend an das proximale Ende des zweiten Konnektorteils ein Ringspalt zwischen dem zweiten Konnektorteil und der eingeführten Vorrichtung gebildet ist, und dass das erste Konnektorteil eine in Richtung auf ein distales Ende offene Ringnut aufweist, in welche im konnektierten Zustand des ersten Konnektorteils und des zweiten Konnektorteils das proximale Ende des zweiten Konnektorteils eintaucht. Eine derartige Anordnung kann eine einfache Konnektierung ermöglichen. Zudem kann durch diese Ausgestaltung die Dichtwirkung verbessert werden.

Vorzugsweise ist der erste Vorsprung auf einer Bodenfläche der Ringnut angeordnet. Durch diese Ausgestaltung kann die zwischen dem ersten Konnektorteil und dem zweiten Konnektorteil gebildete Dichtung gegen äußere Einflüsse geschützt angeordnet werden.

Vorteilhafterweise weist der Kanal der Vorrichtung, insbesondere der Schlauch der Vorrichtung, eine seitliche Öffnung auf, welche insbesondere im konnektierten Zustand des ersten Konnektorteils und des zweiten Konnektorteils in den Ringspalt mündet. Diese Öffnung ermöglicht auf einfache Art und Weise ein Füllen des Ringspalts zwischen dem zweiten Konnektorteil sowie dem daran anschließenden Verweilkanülenrohr einerseits und der eingeführten Vorrichtung andererseits mit Fluid.

Eine besonders bevorzugte Ausführungsform der Erfindung sieht vor, dass das Konnektieren des ersten Konnektorteils und des zweiten Konnektorteils nach Art eines Bajonettverschlusses erfolgt, wobei vorzugsweise das Einrasten des Bajonettverschlusses haptisch und/oder akustisch wahrnehmbar ist. Ein Bajonettverschluss lässt sich schnell und einfach mechanisch schließen und öffnen, indem die beiden Konnektorteile ineinandergesteckt und in entgegengesetzte Richtung gedreht werden. In der geschlossenen Position rastet ein Stift des Bajonettverschlusses in eine Rastaufnahme ein. Der Widerstand des Einrastens kann vorzugsweise so groß gewählt werden, dass es für einen Benutzer haptisch und/oder akustisch, beispielsweise durch ein Klickgeräusch, wahrnehmbar ist, wodurch die Sicherheit der Bedienung erhöht werden kann.

Vorzugsweise ist das Stimulationselement als elektrisch leitenden Stimulationsdraht mit einem Durchmesser, einem distalen Ende und einem proximalen Ende, und der Kanal als ein elektrisch isolierender Schlauch mit einem Innendurchmesser, einem Außendurchmesser, einem distalen Ende und einem proximalen Ende ausgebildet ist, wobei der Stimulationsdraht in dem Schlauch angeordnet ist, wobei der Innendurchmesser des Schlauchs größer ist als der Durchmesser des Stimulationsdrahts, so dass ein Spalt zur Zufuhr von Fluid in dem Schlauch gebildet ist, wobei an dem distalen Ende des Stimulationsdrahts eine Stimulationselektrode elektrisch leitend angeordnet ist, welche über das distale Ende des Schlauchs vorsteht. Dadurch, dass der Stimulationsdraht innerhalb des Lumens des Schlauchs angeordnet ist, kann sich ein besonders kompakter Aufbau ergeben.

Aufgrund der Flexibilität des Schlauchs kann zudem eine langgestreckte, flexible Vorrichtung bereitgestellt werden, die entweder durch Kanülen oder Verweilkanülen in den Körper, aber auch durch eine natürliche Körperöffnung, beispielsweise die Nase, die Blase oder die Vagina, in den Körper ohne das Erfordernis, einen Schnitt in die Haut setzen zu müssen, eingeführt werden kann. Dadurch kann eine Stimulation auch von Nerven erfolgen, die durch außen am Körper auf die Haut geklebte Stimulationselektroden nur schwer oder gar nicht zugänglich sind.

Vorzugsweise steht das proximale Ende des Stimulationsdrahts über das proximale Ende des Schlauchs vor und ist um das proximale Ende des Schlauchs umgebogen auf die Außenseite des Schlauchs geführt, wobei ein elektrisch leitendes Stimulationskabel an dem proximalen Ende des Stimulationsdrahts elektrisch leitend verbunden ist und wobei ein Zuspritzschlauch abschnittsweise über das proximale Ende des Schlauchs abdichtend gestülpt ist. Der am proximalen Ende des Schlauchs umgebogene Stimulationsdraht kann eine einfache Anbindung des Stimulationskabels auf der Außenseite des Schlauchs ermöglichen, ohne den Stimulationsdraht durch die Wandung des Schlauchs führen zu müssen, so dass eine mögliche Undichtigkeitsstelle vermieden werden kann.

Gemäß einer vorteilhaften Weiterbildung der Erfindung weist die Stimulationselektrode eine abgerundete Außenfläche auf und ist beispielsweise kalottenförmig, kugelförmig, ellipsoidförmig, pilzförmig, birnenförmig oder eiförmig ausgebildet. Durch die abgerundete Ausgestaltung können Verletzungen am Körper der zu behandelnden Person vermieden werden. Zudem kann eine derartige Ausgestaltung der Stimulationselektrode das Vorschieben der Vorrichtung, insbesondere in die Körperöffnung, vereinfachen.

Vorzugsweise weist die Stimulationselektrode in jedem Querschnitt einen Außendurchmesser aufweist, welcher kleiner oder gleich ist als der Außendurchmesser des Schlauchs. Mit anderen Worten sind die Außenmaße der Stimulationselektrode nicht größer als die Außenmaße des Schlauchs, so dass die Stimulationselektrode nicht seitlich übersteht. Eine derartige Ausgestaltung kann das Vorschieben der Vorrichtung durch die Verweilkanüle vereinfachen.

Grundsätzlich ist denkbar, dass das Fluid am distalen Ende des Schlauchs zwischen der Stimulationselektrode und der Schlauchwandung austreten kann. Vorteilhafterweise ist die Stimulationselektrode mit einem Befestigungsabschnitt in dem distalen Ende des Schlauchs formschlüssig angeordnet, wobei der Schlauch in seiner Wandung wenigstens eine in den Spalt zwischen Stimulationsdraht und Schlauch mündende Zuspritzöffnung aufweist und/oder die Stimulationselektrode eine axiale Durchgangsöffnung aufweist. Die formschlüssige Anordnung ermöglicht eine Stabilisierung des distalen Endes des Stimulationsdrahts innerhalb des Schlauchs. Weiterhin kann die formschlüssige Anordnung einen Verschluss des distalen Endes des Schlauchs ermöglichen, um ein Eindringen von Partikeln in das distale Ende des Schlauchs oder gar ein Zusetzen des distalen Endes des Schlauchs zu verhindern. Das Fluid kann in dieser Ausführungsform seitlich durch die wenigstens eine in der Wandung des Schlauchs angeordnete Zuspritzöffnung und/oder durch die axiale Durchgangsöffnung der Stimulationselektrode austreten.

Vorzugsweise sind das proximale Ende des Schlauchs mit der Verbindungsstelle zu dem Stimulationskabel und der Verbindungsstelle zu dem Zuspritzschlauch in einem Gehäuse angeordnet, wobei das Stimulationskabel und der Zuspritzschlauch proximal, insbesondere parallel zur Längsachse des Schlauchs, aus dem Gehäuse austreten und das erste Konnektorteil an dem Gehäuse angeordnet ist, insbesondere einstückig mit diesem verbunden ist. Das proximale Ende des Schlauchs einschließlich der Verbindungsstellen kann dabei in das Gehäuse eingelegt und optional zusätzlich mit einem Kleber vergossen oder mit einem Gehäuse umspritzt werden. Auf diese Weise können die Verbindungsstellen geschützt angeordnet werden.

Der Durchmesser des Stimulationsdrahts beträgt vorzugsweise weniger als 0,3 mm, vorzugsweise weniger als 0,2 mm, besonders bevorzugt weniger als 0,1 mm. Auf diese Weise kann ein dünner, flexibler Stimulationsdraht bereitgestellt werden, der ein einfaches Vorschieben in den Körper ermöglichen kann.

Vorzugsweise ist der Stimulationsdraht aus Edelstahl gefertigt. Dadurch kann die erforderliche Stabilität des Stimulationsdrahts erreicht werden, insbesondere auch bei Längen von mehr als 10 cm.

Gemäß einer besonders bevorzugten Ausführungsform der Erfindung beträgt der Außendurchmesser des Schlauchs weniger als 1,0 mm, vorzugsweise weniger als 0,9 mm. Dadurch kann eine Dimensionierung der Vorrichtung zur Nervenstimulation ermöglicht werden, welche ein einfaches Einführen in den Körper ermöglichen kann.

Vorzugsweise liegt der Innendurchmesser des Schlauchs im Bereich von 0,4 mm bis 0,7 mm, vorzugsweise im Bereich von 0,5 mm bis 0,6 mm. Eine derartige Dimensionierung ermöglicht einen ausreichend großen Spalt zwischen Innenwandung des Schlauchs und Stimulationsdraht, um ein Fluid durch den Schlauch vom proximalen bis zum distalen Ende führen zu können.

Gemäß einer bevorzugten Weiterbildung der Erfindung beträgt die Länge des Schlauchs mehr als 5 cm, vorzugsweise mehr als 8 cm, besonders bevorzugt mehr als 10 cm. Dadurch wird eine genügende Länge bereitgestellt, um die Vorrichtung zur Nervenstimulation ausreichend weit in den Körper einschieben zu können.

Eine bevorzugte Ausführungsform der Erfindung sieht vor, dass der Schlauch aus Kunststoff, beispielsweise einem Polyamid, gefertigt ist. Ein derartiges Material kann eine ausreichend Dichtigkeit, eine hohe Flexibilität sowie eine gute Biokompatibilität ermöglichen.

Eine bevorzugte Weiterbildung der Erfindung sieht vor, dass das Set zusätzlich eine starre Kanüle mit einem Kanülenrohr mit einem distalen Ende und einem proximalen Ende zum Einstich in einen Körper umfasst, auf welche die Verweilkanüle vor dem Einstich aufschiebbar ist und welche an ihrem proximalen Ende ein zu dem zweiten Konnektorteil komplementäres drittes Konnektorteil aufweist, wobei durch Konnektieren des dritten Konnektorteils und des zweiten Konnektorteils die Kanüle in einer axialen Position festlegbar ist, in welcher das distale Ende des Kanülenrohrs über das distale Ende der Verweilkanüle hervorragt, wobei das dritte Konnektorteil und das zweite Konnektorteil derart miteinander verbunden sind, dass eine umlaufende zweite Dichtung gebildet ist. Die starre Kanüle ermöglicht ein Einführen der Verweilkanüle durch die Haut in den Körper, um auch Nerven stimulieren zu können, die bei Stimulation durch die Haut nicht zugänglich sind.

Vorteilhafterweise ist die zweite Dichtung durch Anlage einer dritten Kontaktfläche des dritten Konnektorteils an der zweiten Kontaktfläche des zweiten Konnektorteils gebildet, wodurch zusätzliche Komponenten wie beispielsweise ein Dichtungsring vermieden werden können.

Gemäß einer bevorzugten Ausführungsform der Erfindung ist vorgesehen, dass das dritte Konnektorteil eine distale Fläche aufweist, auf welcher ein in axialer Richtung vorstehender umlaufender dritter Vorsprung angeordnet ist, und die dritte Kontaktfläche auf der Außenseite des dritten Vorsprungs und die zweite Kontaktfläche auf der Innenseite des zweiten Vorsprungs oder alternativ die dritte Kontaktfläche auf der Innenseite des dritten Vorsprungs und die zweite Kontaktfläche auf der Außenseite des zweiten Vorsprungs angeordnet ist. Auf diese Weise kann sich über einen in axialer Richtung verlaufenden Abschnitt eine umlaufende Anlagefläche zwischen der dritten Kontaktfläche und der zweiten Kontaktfläche ergeben, welche eine zuverlässige Bildung der zweiten Dichtung ermöglicht.

Vorzugsweise weist die dritte Kontaktfläche einen in Richtung auf die zweite Kontaktfläche vorstehenden umlaufenden Wulst und/oder die zweite Kontaktfläche einen in Richtung auf die dritte Kontaktfläche vorstehenden umlaufenden Wulst auf, welcher insbesondere bewirkt, dass der dritte Vorsprung und der zweite Vorsprung unter Vorspannung gegeneinander anliegen, wodurch die Dichtwirkung verbessert werden kann.

Vorteilhafterweise weist das dritte Konnektorteil eine in Richtung auf ein distales Ende offene Ringnut auf, in welche im konnektierten Zustand des dritten Konnektorteils und des zweiten Konnektorteils das proximale Ende des zweiten Konnektorteils eintaucht. Eine derartige Anordnung kann eine einfache Konnektierung ermöglichen. Zudem kann durch diese Ausgestaltung die Dichtwirkung verbessert werden.

Bevorzugt ist der dritte Vorsprung auf einer Bodenfläche der Ringnut angeordnet. Durch diese Ausgestaltung kann die zwischen dem dritten Konnektorteil und dem zweiten Konnektorteil gebildete Dichtung gegen äußere Einflüsse geschützt angeordnet werden.

Vorzugsweise weist das Kanülenrohr eine seitliche Öffnung auf, welche insbesondere im konnektierten Zustand des dritten Konnektorteils und des zweiten Konnektorteils in den Ringspalt mündet. Diese Öffnung ermöglicht auf einfache Art und Weise ein Füllen des Ringspalts zwischen dem zweiten Konnektorteil sowie dem daran anschließenden Verweilkanülenrohr einerseits und der eingeführten Kanüle andererseits mit Fluid. Dadurch kann insbesondere beim Einstechen der Kanüle in den Körper die Ultraschallsichtbarkeit der Kanüle verbessert werden.

Gemäß einer vorteilhaften Weiterbildung erfolgt das Konnektieren des dritten Konnektorteils und des zweiten Konnektorteils nach Art eines Bajonettverschlusses, wobei vorzugsweise das Einrasten des Bajonettverschlusses haptisch und/oder akustisch wahrnehmbar ist. Ein Bajonettverschluss lässt sich schnell und einfach mechanisch schließen und öffnen, indem die beiden Konnektorteile ineinandergesteckt und in entgegengesetzte Richtung gedreht werden. In der geschlossenen Position rastet ein Stift des Bajonettverschlusses in eine Rastaufnahme ein.

Der Widerstand des Einrastens kann vorzugsweise so groß gewählt werden, dass es für einen Benutzer haptisch und/oder akustisch, beispielsweise durch ein Klickgeräusch, wahrnehmbar ist, wodurch die Sicherheit der Bedienung erhöht werden kann.

Besonders bevorzugt ist das dritte Konnektorteil im Wesentlichen identisch zu dem ersten Konnektorteil ausgebildet.

Vorteilhafterweise ist das Kanülenrohr aus einem elektrisch leitenden Material gefertigt und ein elektrisch leitendes Stimulationskabel auf der Außenseite des Kanülenrohrs elektrisch leitend verbunden, wobei ein Zuspritzschlauch abschnittsweise über das proximale Ende des Kanülenrohrs abdichtend gestülpt ist. Die Kanüle eignet sich damit sowohl zur Nervenstimulation als auch zur Zufuhr eines Fluids, beispielsweise einer Flüssigkeit wie eines Anästhetikums, und kann dabei kompakt aufgebaut sein.

Vorzugsweise ist das proximale Ende des Kanülenrohrs mit der Verbindungsstelle zu dem Stimulationskabel und der Verbindungsstelle zu dem Zuspritzschlauch in einem Gehäuse angeordnet sind, wobei das Stimulationskabel und der Zuspritzschlauch proximal, insbesondere parallel zur Längsachse des Kanülenrohrs, aus dem Gehäuse herausgeführt sind, und das dritte Konnektorteil an dem Gehäuse angeordnet ist, insbesondere einstückig mit diesem verbunden ist. Das proximale Ende des Schlauchs einschließlich der Verbindungsstellen kann dabei in das Gehäuse eingelegt und optional zusätzlich mit einem Kleber vergossen oder mit einem Gehäuse umspritzt werden. Auf diese Weise können die Verbindungsstellen geschützt angeordnet werden.

Ein Ausführungsbeispiel der Erfindung wird anhand der nachfolgenden Figuren ausführlich erläutert. Es zeigen
- Fig. 1a: eine Seitenansicht eines Set zur Nervenstimulation mit einer Vorrichtung zur Nervenstimulation mit einem ersten Konnektorteil und einer Verweilkanüle mit einem zweiten Konnektorteil, wobei die Vorrichtung in der Verweilkanüle angeordnet ist,
- Fig. 1b: die Vorrichtung zur Nervenstimulation gemäß Fig. 1a ohne Verweilkanüle,
- Fig. 2: einen Längsschnitt durch die Vorrichtung gemäß Fig. 1b,
- Fig. 3: eine Ausschnittvergrößerung aus Fig. 2 zur Darstellung der elektrischen Anbindung des Stimulationsdrahts der Vorrichtung zur Nervenstimulation,
- Fig. 4: eine Ausschnittvergrößerung des distalen Endes der Vorrichtung aus Fig. 2,
- Fig. 5: eine Ausschnittvergrößerung aus Fig. 3 zur Darstellung eines Biegeabschnitts des Stimulationsdrahts,
- Fig. 6: einen Ausschnitt eines Längsschnitts durch die in der Verweilkanüle angeordnete Vorrichtung gemäß Fig. 1,
- Fig. 7: eine Ausschnittvergrößerung aus Fig. 6 mit konnektiertem ersten Konnektorteil und zweiten Konnektorteil,
- Fig. 8: eine Ansicht vergleichbar zu Fig. 7, wobei das erste Konnektorteil und das zweite Konnektorteil gelöst sind,
- Fig. 9: eine weitere Ausschnittvergrößerung aus Fig. 6 zur Darstellung einer Öffnung des Schlauchs der Vorrichtung zur Nervenstimulation,
- Fig. 10: einen Längsschnitt durch die Verweilkanüle aus Fig. 1, in welcher eine Kanüle mit einem dritten Konnektorteil angeordnet ist,
- Fig. 11: eine Ausschnittvergrößerung aus Fig. 10 mit konnektiertem dritten Konnektorteil und zweiten Konnektorteil,
- Fig. 12: eine Ansicht vergleichbar zu Fig. 11, wobei das dritte Konnektorteil und das zweite Konnektorteil gelöst sind,
- Fig. 13: eine Ausschnittvergrößerung aus Fig. 10 zur Darstellung der elektrischen Anbindung der Kanüle,
- Fig. 14: eine weitere Ausschnittvergrößerung aus Fig. 10 zur Darstellung einer seitlichen Öffnung in dem Kanülenrohr der Kanüle und
- Fig. 15: eine Draufsicht auf die distalen Enden der Vorrichtung zur Nervenstimulation gemäß Fig. 1b und der Kanüle gemäß Fig. 10.

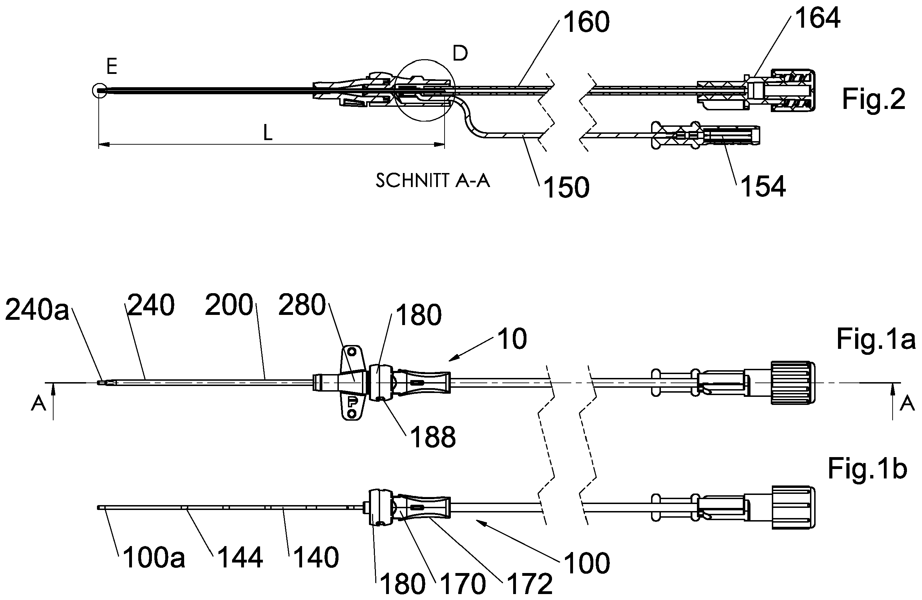

Die Figuren 1 bis 15 zeigen verschiedene Ansichten eines Ausführungsbeispiels eines Sets 10 zur Nervenstimulation. Zur besseren Übersicht sind nicht sämtliche Bezugsziffern in sämtlichen Figuren angegeben.

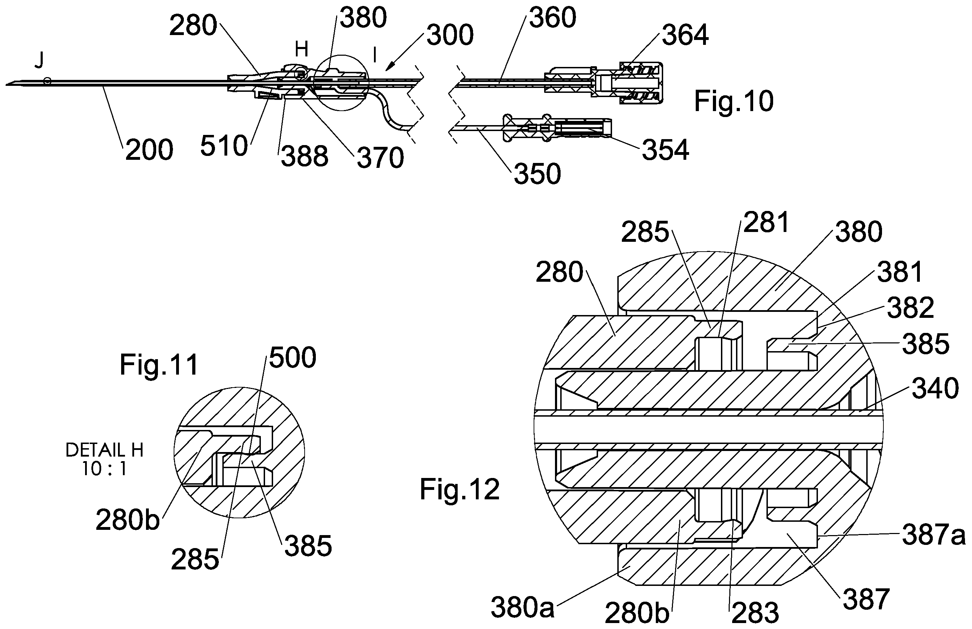

Das Set 10 zur Nervenstimulation umfasst zumindest eine Vorrichtung 100 zur Nervenstimulation und eine Verweilkanüle 200, welche anhand der Figuren 1 bis 9 näher erläutert werden, und kann optional zusätzlich eine Kanüle 300 umfassen, welche anhand der Figuren 10 bis 15 näher erläutert wird.

Die Vorrichtung 100 umfasst ein elektrisch leitendes Stimulationselement, welches insbesondere als ein elektrisch leitender Stimulationsdraht 120 mit einem Durchmesser AD, einem distalen Ende 120a und einem proximalen Ende 120b ausgebildet ist. Bei dem Stimulationsdraht 120 kann es sich um einen flexiblen, biegbaren Draht handeln. Der Stimulationsdraht 120 ist beispielsweise aus Edelstahl gefertigt. Der Durchmesser AD des Stimulationsdrahts 20 kann weniger als 0,3 mm, vorzugsweise weniger als 0,2 mm, besonders bevorzugt weniger als 0,1 mm, beispielsweise etwa 0,07 mm, betragen.

Weiterhin umfasst die Vorrichtung 100 einen Kanal zur Zufuhr eines Fluids, welcher insbesondere als ein elektrisch isolierender Schlauch 140 mit einem Innendurchmesser IS, einem Außendurchmesser AS, einem distalen Ende 140a und einem proximalen Ende 140b ausgebildet ist. Bei dem Schlauch 140 handelt es sich insbesondere um einen flexiblen, dichten und biokompatiblen Schlauch 140. Der Schlauch 140 kann beispielsweise aus Kunststoff, vorzugsweise einem Polyamid, gefertigt sein. Der Außendurchmesser AS des Schlauchs 140 beträgt vorzugsweise weniger als 1,0 mm, besonders bevorzugt weniger als 0,9 mm, und kann beispielsweise 0,87 mm betragen. Der Innendurchmesser IS des Schlauchs 140 ist größer als der Durchmesser AD des Stimulationsdrahts 120 und kann im Bereich von 0,4 mm bis 0,7 mm, vorzugsweise im Bereich von 0,5 mm bis 0,6 mm, liegen und beispielsweise 0,54 mm betragen. Die Länge L des Schlauchs 140 beträgt mehr als 5 cm, vorzugsweise mehr als 8 cm, beispielsweise 8,35cm, und besonders bevorzugt mehr als 10 cm, beispielsweise 11,35 oder 16,35 cm betragen. Ausgehend von dem distalen Ende 140a kann der Schlauch 140 Markierungen 144 aufweisen (siehe Fig. 1b), welche insbesondere äquidistant angeordnet sind und dem Benutzer Informationen darüber geben können, um welche Länge das distale Ende 140a des Schlauchs 140 in einen Körper eingeführt wurde.

Der Stimulationsdraht 120 ist insbesondere in dem Schlauch 140 angeordnet, insbesondere durch das Lumen des Schlauchs 140 geführt. Dadurch, dass der Innendurchmesser IS des Schlauchs 140 größer ist als der Durchmesser AD des Stimulationsdrahts 120, wird dabei ein Spalt 146 in dem Schlauch 140, gebildet, durch welchen wie nachfolgend näher beschrieben Flüssigkeit vom proximalen Ende 140b des Schlauchs 140 zum distalen Ende 140a des Schlauchs 140 geführt werden kann. Der Spalt 146 kann nach Art eines Ringspalts zwischen dem Stimulationsdraht 120 und der Innenwandung des Schlauchs 140 ausgebildet sein, wenn der Stimulationsdraht 120 im Wesentlichen zentrisch in dem Lumen angeordnet ist. Alternativ kann der Stimulationsdraht 120 auch an der Innenwandung des Schlauchs 140 anliegen, so dass der Spalt 146 sichelförmig ausgebildet sein kann.

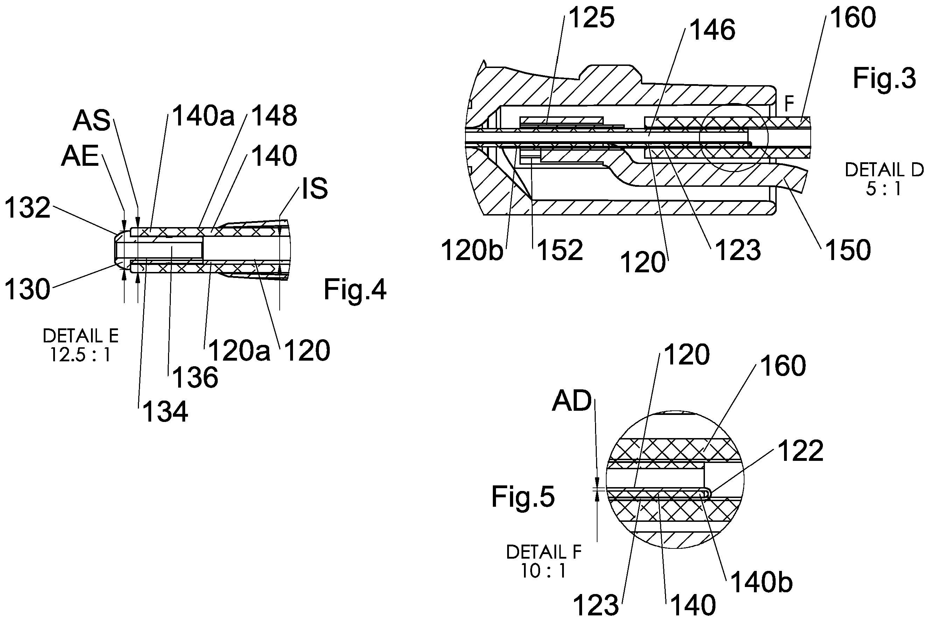

An dem distalen Ende 120a des Stimulationsdrahts 120 ist eine Stimulationselektrode 130 elektrisch leitend angeordnet, welche über das distale Ende 140a des Schlauchs 140 vorsteht. Die Stimulationselektrode 130 kann eine abgerundete Außenfläche 132 aufweisen und beispielsweise kalottenförmig, kugelförmig, ellipsoidförmig, pilzförmig, birnenförmig oder eiförmig oder in sonstiger Weise ballig ausgebildet sein. Durch die konvexe Ausgestaltung der Stimulationselektrode 130 weist diese in wenigstens einem Querschnitt einen größten Außendurchmesser AE auf. Dieser Außendurchmesser AE der Stimulationselektrode 130 ist jedoch höchstens so groß wie der Außendurchmesser AS des Schlauchs 140 (vgl. insbesondere Fig. 4).

Die elektrisch leitende Verbindung zwischen dem Stimulationsdraht 120 und der Stimulationselektrode 130 kann beispielsweise dadurch gebildet sein, dass das distale Ende 120a des Stimulationsdrahts 120 an einem an der Stimulationselektrode 130 angeordneten Befestigungsabschnitt 134 durch Verschweißen fixiert oder zwischen dem Befestigungsabschnitt 134 und dem Schlauch 140 verklemmt wird (siehe Figur 4).

Die Stimulationselektrode 130 kann mit dem Befestigungsabschnitt 134 in dem distalen Ende 140a des Schlauchs 140, genauer gesagt in einem von dem distalen Ende 140a ausgehenden Abschnitt 148 des Schlauchs 140, formschlüssig angeordnet sein. Der Befestigungsabschnitt 134 kann auf seiner Außenseite eine oder mehrere Rillen aufweisen, welche eine klemmende Fixierung des Befestigungsabschnitts 134 in dem Abschnitt 148 des Schlauchs 140 verbessern. Die Stimulationselektrode 130, insbesondere der Befestigungsabschnitt 134, kann dadurch das distale Ende 140a des Schlauchs 140 im Wesentlichen ausfüllen. Um ein Austreten eines in dem Spalt 146 angeordneten Fluids am distalen Ende 100a der Vorrichtung 100 ermöglichen zu können, kann der Schlauch 140 in seiner Wandung wenigstens eine in den Spalt 146 zwischen dem Stimulationsdraht 120 und dem Schlauch 140 mündende Zuspritzöffnung (nicht dargestellt) und/oder die Stimulationselektrode 130 eine axiale Durchgangsöffnung (136) aufweisen.

Das proximale Ende 120b des Stimulationsdrahts 120 steht über das proximale Ende 140b des Schlauchs 140 vor und ist, insbesondere in einem Biegeabschnitt 122, um das proximale Ende 140b des Schlauchs 140 umgebogen, insbesondere um etwa 180°, auf die Außenseite des Schlauchs 140 geführt (vgl. Fig. 3 und 5). Ein elektrisch leitendes Stimulationskabel 150, insbesondere eine elektrisch leitende Ader 152 des Stimulationskabels 150, ist an dem proximalen Ende 120b des Stimulationsdrahts 120, welcher insbesondere mit einem sich an den Biegeabschnitt 122 anschließenden zurückgeführten Abschnitt 123 auf der Außenseite des Schlauchs 140 zu liegen kommt, elektrisch leitend verbunden. Die elektrisch leitende Kontaktierung des Stimulationsdrahts 120 kann somit außerhalb des bei Verwendung oft flüssigkeitsgefüllten Lumens des Schlauchs 140 und ohne Durchbruch durch die Wandung des Schlauchs 140 erfolgen. Das Stimulationskabel 150, insbesondere die Ader 152 des Stimulationskabels 150, kann beispielsweise mittels einer Crimphülse 125 elektrisch leitend mit dem Stimulationsdraht 120, insbesondere dem zurückgeführten Abschnitt 123, verbunden werden (siehe insbesondere Fig. 3). Wie insbesondere in Figur 2 erkennbar, kann am anderen Ende des Stimulationskabels 150 ein elektrischer Steckanschluss 154 zum Anschluss eines Steuergeräts mit Pulsgenerator angeordnet sein.

Ein Zuspritzschlauch 160 kann abschnittsweise über das proximale Ende 140b des Schlauchs 140, und dabei insbesondere abschnittsweise auch über den an der Außenseite des Schlauchs 140 liegenden zurückgeführten Abschnitt 123 des Stimulationsdrahts 120, abdichtend gestülpt sein. Dies kann beispielsweise dadurch erreicht werden, dass ein Innendurchmesser des Zuspritzschlauchs 160 in etwa dem Außendurchmesser AS des Schlauchs 140 entspricht. Wie insbesondere in Figur 2 erkennbar, kann am anderen Ende des Zuspritzschlauchs 160 ein Spritzenanschluss 164 angeordnet sein, über welchen beispielsweise mit einer Spritze ein Fluid, beispielsweise ein Anästhetikum, in den Zuspritzschlauch 160 eingespritzt werden kann, welches über den Zuspritzschlauch 160 in das proximale Ende 140b des Schlauchs 140 eintritt und am distalen Ende 140a des Schlauchs 140, insbesondere durch die axiale Durchgangsöffnung 136 der Stimulationselektrode 130, austritt.

Das proximale Ende 140b des Schlauchs 140 mit der Verbindungsstelle zu dem Stimulationskabel 150 und der Verbindungsstelle zu dem Zuspritzschlauch 160 kann in einem Gehäuse 170 angeordnet sein, wobei das Stimulationskabel 150 und der Zuspritzschlauch 160 proximal, insbesondere parallel zur Längsachse des Schlauchs 140, aus dem Gehäuse 170 austreten. Das proximale Ende 140b des Schlauchs 140 einschließlich der Verbindungsstellen kann dabei in das Gehäuse 170 eingelegt und zusätzlich mit einem Kleber vergossen oder alternativ mit dem Gehäuse 170 umspritzt werden. Auf diese Weise können die Verbindungsstellen geschützt angeordnet werden. An dem Gehäuse 170 können ein oder mehrere Griffmulden 172 zum Einlegen der Finger eines Benutzers angeordnet sein, um die Griffsicherheit zu erhöhen.

Die Verweilkanüle 200 umfasst ein Verweilkanülenrohr 240 mit einem Innendurchmesser IV, einem Außendurchmesser AV, einem distalen Ende 240a und einem proximalen Ende 240b. Das Verweilkanülenrohr 240 kann aus Kunststoff, vorzugsweise einem Polyurethan, gefertigt sein und vorzugsweise aus einem elektrisch isolierenden Material gefertigt sein. Besonders vorteilhaft können in das Verweilkanülenrohr 240 Röntgenkontraststreifen integriert sein. Der Innendurchmesser IV ist größer als der Außendurchmesser AS des Schlauchs 140 der Vorrichtung 100, damit ein Einführen des Schlauchs 140 der Vorrichtung 100 in die Verweilkanüle 200 möglich ist. Insbesondere weist das Verweilkanülenrohr 240 eine höhere Biegesteifigkeit auf als der Schlauch 140.

Zur relativen Lagefixierung umfasst die Vorrichtung 100 ein erstes Konnektorteil 180, während die Verweilkanüle ein zu dem ersten Konnektorteil 180 komplementäres zweites Konnektorteil 280 aufweist, wobei durch Konnektieren des ersten Konnektorteils 180 und des zweiten Konnektorteils 280 die Vorrichtung 100 in einer axialen Position festlegbar ist, in welcher das distale Ende 100a der Vorrichtung 100 über das distale Ende 240a der Verweilkanüle 240 hervorragt. Das erste Konnektorteil 180 kann in der Nähe zum proximalen Ende 140b des Schlauchs 140 der Vorrichtung 100 angeordnet sein, während das zweite Konnektorteil 280 in der Nähe zum proximalen Ende 240b des Verweilkanülenrohrs 240 der Verweilkanüle 100 angeordnet sein kann. Insbesondere kann das erste Konnektorteil 180 an dem Gehäuse 170 der Vorrichtung 100 angeordnet sein und vorzugsweise einstückig mit diesem verbunden sein.

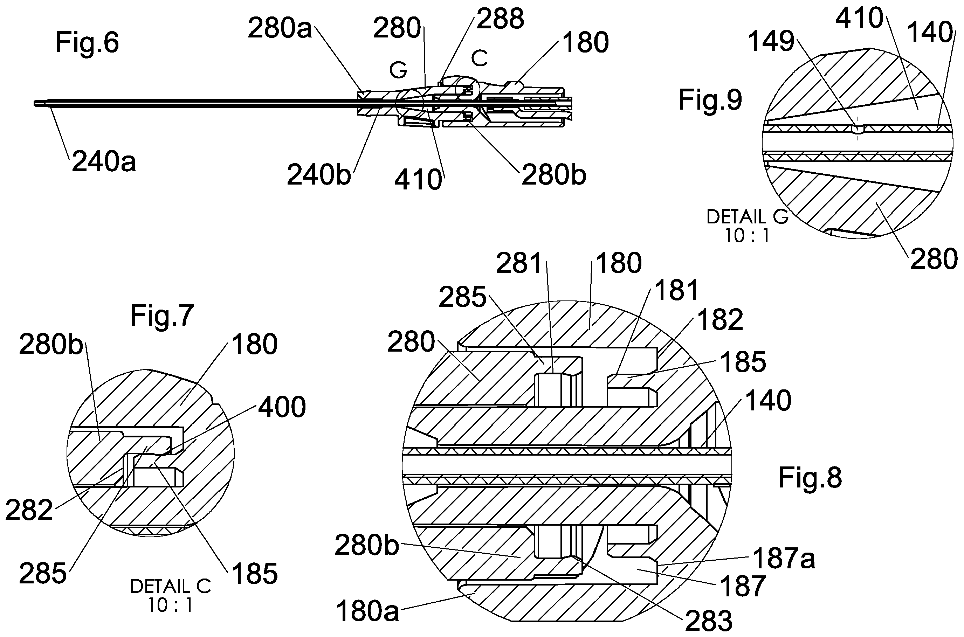

Im konnektierten Zustand des ersten Konnektorteils 180 und des zweiten Konnektorteils 280 sind das erste Konnektorteil 180 und das zweite Konnektorteil 280 derart miteinander verbunden sind, dass eine umlaufende erste Dichtung 400 gebildet ist (vgl. Fig. 7). Dabei kann die erste Dichtung 400 beispielsweise durch Anlage einer ersten Kontaktfläche 181 des ersten Konnektorteils 180 an einer zweiten Kontaktfläche 281 des zweiten Konnektorteils 280 gebildet sein.

Das erste Konnektorteil 180 kann eine distale Fläche 182 aufweisen, auf welcher ein in axialer Richtung vorstehender umlaufender erster Vorsprung 185 angeordnet ist. Das zweite Konnektorteil 280 kann eine proximale Fläche 282 aufweisen, auf welcher ein in axialer Richtung vorstehender umlaufender zweiter Vorsprung 285 angeordnet ist. Wie in den Figuren 7 und 8 dargestellt, sind die erste Kontaktfläche 181 auf der Außenseite des ersten Vorsprungs 185 und die zweite Kontaktfläche 281 auf der Innenseite des zweiten Vorsprungs 285 angeordnet. In einer alternativen, nicht dargestellten Ausführungsform ist auch denkbar, dass die erste Kontaktfläche 181 auf der Innenseite des ersten Vorsprungs 185 und die zweite Kontaktfläche 281 auf der Außenseite des zweiten Vorsprungs 285 angeordnet ist. Die beiden Vorsprünge 185, 285 sind in beiden Ausführungsformen insbesondere derart zueinander angeordnet, dass ihre Kontaktflächen 181, 182 aneinander vorbeigleiten, wenn die beiden Konnektorteile 180, 280 konnektiert werden.

Wie insbesondere in Figur 8 erkennbar, kann die zweite Kontaktfläche 281 einen in Richtung auf die erste Kontaktfläche 181 vorstehenden umlaufenden Wulst 283 aufweisen. Alternativ oder zusätzlich kann die erste Kontaktfläche 181 einen in Richtung auf die zweite Kontaktfläche 281 vorstehenden umlaufenden Wulst aufweisen (nicht dargestellt).

Das zweite Konnektorteil 280 kann an einem distalen Ende 280a das Verweilkanülenrohr 240, insbesondere das proximale Ende 240b des Verweilkanülenrohrs 240, abdichtend umschließen und sich in Richtung auf ein proximales Ende 280b aufweiten. Dadurch ist bei in die Verweilkanüle 200 eingeführter Vorrichtung 100 angrenzend an das proximale Ende 280b des zweiten Konnektorteils 280 ein Ringspalt 410 zwischen dem zweiten Konnektorteil 280 und der eingeführten Vorrichtung 100 gebildet (vgl. insbesondere Fig. 6 und 9). Der Ringspalt 410 verjüngt sich insbesondere in distaler Richtung (vgl. Fig. 9).

Der Kanal der Vorrichtung 100, insbesondere der Schlauch 140 der Vorrichtung 100, kann eine seitliche Öffnung 149 aufweisen, welche insbesondere im konnektierten Zustand des ersten Konnektorteils 180 und des zweiten Konnektorteils 280 in den Ringspalt 410 mündet.

Das erste Konnektorteil 180 kann eine in Richtung auf ein distales Ende 180a des ersten Konnektorteils 180 offene Ringnut 187 aufweisen, in welche im konnektierten Zustand des ersten Konnektorteils 180 und des zweiten Konnektorteils 280 das proximale Ende 280b des zweiten Konnektorteils 280 eintaucht (vgl. insbesondere Fig. 7 und 8. Der erste Vorsprung 185 kann dabei auf einer Bodenfläche 187a der Ringnut 187 angeordnet sein.

Das Konnektieren des ersten Konnektorteils 180 und des zweiten Konnektorteils 280 kann nach Art eines Bajonettverschlusses erfolgen, wobei insbesondere zum Verbinden das proximale Ende 280b des zweiten Konnektorteils 280 in die Ringnut 187 des ersten Konnektorteils 180 eingeschoben wird und anschließend die beiden Konnektorteile 180, 280 gegensinnig gegeneinander verdreht werden. Dabei kann ein Stift 288 des zweiten Konnektorteils 280 in einer Nut 188 des ersten Konnektorteils 180 geführt sein. Das Einrasten des Bajonettverschlusses, insbesondere des Stifts 288 am Ende der Nut 188 in eine entsprechende Ausnehmung, ist vorzugsweise haptisch und/oder akustisch wahrnehmbar.

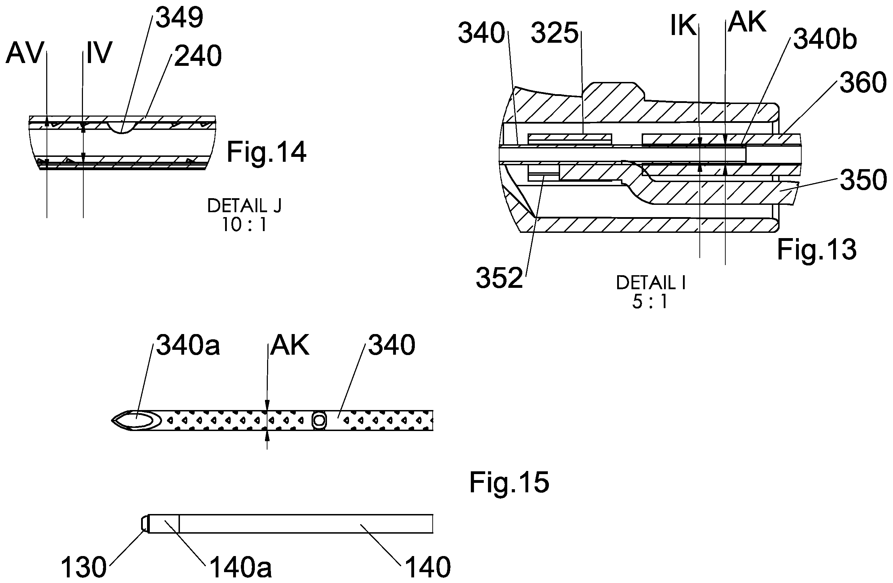

Das Set 10 kann weiterhin eine starre Kanüle 300 mit einem Kanülenrohr 340 mit einem distalen Ende 340a und einem proximalen Ende 340b sowie einem Außendurchmesser AK und einen Innendurchmesser IK umfassen, wobei das distale Ende 340a zum Einstich in einen Körper insbesondere eine Spitze aufweist (siehe insbesondere Fig. 15). Das Kanülenrohr 340 kann aus einem elektrisch leitenden Material, beispielsweise Edelstahl, gefertigt und auf der Außenseite abgesehen von dem distalen Ende 340a und einer Anbindungsstelle zu einem Stimulationskabel 360 isolierend beschichtet sein. Der Außendurchmesser AK des Kanülenrohrs 340 ist kleiner als der Innendurchmesser IV des Verweilkanülenrohrs 240, so dass das Verweilkanülenrohr 240 auf das Kanülenrohr 340 vor dem Einstich aufschiebbar ist. Auf der Außenseite des Kanülenrohrs 340 können ultraschallsichtbarkeitserhöhende Prägungen angebracht sein.

Der Außendurchmesser AK des Kanülenrohrs 340 beträgt vorzugsweise weniger als 1,0 mm, und kann beispielsweise 0,95 mm betragen. Der Innendurchmesser IK des Kanülenrohrs 340 kann im Bereich von 0,4 mm bis 0,7 mm liegen und beispielsweise 0,65 mm betragen.

Die elektrisch leitende Kontaktierung des Kanülenrohrs 340 kann direkt auf der Außenwandung des Kanülenrohrs 340 erfolgen. Dazu kann ein Stimulationskabel 350, insbesondere eine Ader 352 des Stimulationskabels 350, beispielsweise mittels einer Crimphülse 325 elektrisch leitend mit dem Kanülenrohr 340 verbunden werden (siehe insbesondere Fig. 13). Wie insbesondere in Figur 10 erkennbar, kann am anderen Ende des Stimulationskabels 350 ein elektrischer Steckanschluss 354 zum Anschluss eines Steuergeräts mit Pulsgenerator angeordnet sein.

Ein Zuspritzschlauch 360 kann abschnittsweise über das proximale Ende 340b des Kanülenrohrs 340 abdichtend gestülpt sein (siehe Fig. 13). Dies kann beispielsweise dadurch erreicht werden, dass ein Innendurchmesser des Zuspritzschlauchs 360 in etwa dem Außendurchmesser AK des Kanülenrohrs 340 entspricht. Wie insbesondere in Figur 10 erkennbar, kann am anderen Ende des Zuspritzschlauchs 360 ein Spritzenanschluss 364 angeordnet sein, über welchen beispielsweise mit einer Spritze ein Fluid, beispielsweise ein Anästhetikum, in den Zuspritzschlauch 360 eingespritzt werden kann, welches über den Zuspritzschlauch 360 in das proximale Ende 340b des Kanülenrohrs 340 eintritt und am distalen Ende 340a des Kanülenrohrs 340, insbesondere durch die angeschliffene Spitze, austritt.

Das proximale Ende 340b des Kanülenrohrs 340 mit der Verbindungsstelle zu dem Stimulationskabel 350 und der Verbindungsstelle zu dem Zuspritzschlauch 360 kann in einem Gehäuse 370 angeordnet sein, wobei das Stimulationskabel 350 und der Zuspritzschlauch 360 proximal, insbesondere parallel zur Längsachse des Schlauchs 340, aus dem Gehäuse 370 austreten (siehe Figur 10). Das proximale Ende 340b des Kanülenrohrs 340 einschließlich der Verbindungsstellen kann dabei in das Gehäuse 370 eingelegt und zusätzlich mit einem Kleber vergossen oder alternativ mit dem Gehäuse 370 umspritzt werden. Auf diese Weise können die Verbindungsstellen geschützt angeordnet werden. An dem Gehäuse 370 können ein oder mehrere Griffmulden zum Einlegen der Finger eines Benutzers angeordnet sein, um die Griffsicherheit zu erhöhen.

Die Kanüle 300 umfasst ein drittes Konnektorteil 380, welches komplementär zu dem zweiten Konnektorteil 280 der Verweilkanüle ausgebildet ist und eine relative Lagepositionierung der Kanüle 300 und der Verweilkanüle 200 ermöglicht. Durch Konnektieren des dritten Konnektorteils 380 und des zweiten Konnektorteils 280 ist die Kanüle 300 in einer axialen Position festlegbar, in welcher das distale Ende 340a des Kanülenrohrs 340 über das distale Ende 240a der Verweilkanüle 240 hervorragt. Das dritte Konnektorteil 380 kann in der Nähe zum proximalen Ende 340b des Kanülenrohrs 340 der Kanüle 300 angeordnet sein. Insbesondere kann das dritte Konnektorteil 380 an dem Gehäuse 370 der Kanüle 300 angeordnet sein und vorzugsweise einstückig mit diesem verbunden sein.

Das dritte Konnektorteil 380 kann im Wesentlichen in seinen funktionellen Merkmalen dem ersten Konnektorteil 180 entsprechen. Dadurch kann insbesondere sowohl die Kanüle 300 als auch alternativ die Vorrichtung 100 zur Nervenstimulation wie nachfolgend noch erläutert in der Verweilkanüle fixiert werden.

Im konnektierten Zustand des dritten Konnektorteils 380 und des zweiten Konnektorteils 280 sind das dritte Konnektorteil 380 und das zweite Konnektorteil 280 derart miteinander verbunden sind, dass eine umlaufende zweite Dichtung 500 gebildet ist (vgl. Fig. 11). Dabei kann die zweite Dichtung 500 beispielsweise durch Anlage einer dritten Kontaktfläche 381 des dritten Konnektorteils 380 an der zweiten Kontaktfläche 281 des zweiten Konnektorteils 280 gebildet sein.

Das dritte Konnektorteil 380 kann eine distale Fläche 382 aufweisen, auf welcher ein in axialer Richtung vorstehender umlaufender dritter Vorsprung 385 angeordnet ist. Wie in den Figuren 11 und 12 dargestellt, ist die dritte Kontaktfläche 381 auf der Außenseite des dritten Vorsprungs 385 und die zweite Kontaktfläche 281 auf der Innenseite des zweiten Vorsprungs 285 angeordnet. In einer alternativen, nicht dargestellten Ausführungsform ist auch denkbar, dass die dritte Kontaktfläche 381 auf der Innenseite des dritten Vorsprungs 385 und die zweite Kontaktfläche 281 auf der Außenseite des zweiten Vorsprungs 285 angeordnet ist. Die beiden Vorsprünge 385, 285 sind in beiden Ausführungsformen insbesondere derart zueinander angeordnet, dass ihre Kontaktflächen 281, 382 aneinander vorbeigleiten, wenn die beiden Konnektorteile 280, 380 konnektiert werden.

Wie insbesondere in Figur 12 erkennbar und anhand von Figur 8 bereits erläutert, kann die zweite Kontaktfläche 281 den in Richtung auf die zweite Kontaktfläche 381 vorstehenden umlaufenden Wulst 283 aufweisen. Alternativ oder zusätzlich kann die dritte Kontaktfläche 381 einen in Richtung auf die zweite Kontaktfläche 281 vorstehenden umlaufenden Wulst aufweisen (nicht dargestellt).

Wie bereits erläutert, kann das zweite Konnektorteil 280 an einem distalen Ende 280a das Verweilkanülenrohr 240, insbesondere das proximale Ende 240b des Verweilkanülenrohrs 240, abdichtend umschließen und sich in Richtung auf das proximale Ende 280b aufweiten. Dadurch ist auch bei in die Verweilkanüle 200 eingeführter Kanüle 300 angrenzend an das proximale Ende 280b des zweiten Konnektorteils 280 ein Ringspalt 510 zwischen dem zweiten Konnektorteil 280 und der eingeführten Kanüle 300 gebildet (vgl. insbesondere Fig. 10). Der Ringspalt 510 verjüngt sich insbesondere in distaler Richtung und kann sich insbesondere entlang des Verweilkanülenrohrs 240 bis zu dessen distalem Ende 240a erstrecken.

Das Kanülenrohr 340 kann eine seitliche Öffnung 349 aufweisen, welche insbesondere im konnektierten Zustand des dritten Konnektorteils 380 und des zweiten Konnektorteils 280 in den Ringspalt 510 mündet (vgl. Fig. 10 und 14).

Das dritte Konnektorteil 380 kann eine in Richtung auf ein distales Ende 380a des dritten Konnektorteils 380 offene Ringnut 387 aufweisen, in welche im konnektierten Zustand des dritten Konnektorteils 380 und des zweiten Konnektorteils 280 das proximale Ende 280b des zweiten Konnektorteils 280 eintaucht (vgl. insbesondere Fig. 11 und 12). Der dritte Vorsprung 385 kann dabei auf einer Bodenfläche 387a der Ringnut 387 angeordnet sein.

Das Konnektieren des dritten Konnektorteils 380 und des zweiten Konnektorteils 280 kann nach Art eines Bajonettverschlusses erfolgen, wobei insbesondere zum Verbinden das proximale Ende 280b des zweiten Konnektorteils 280 in die Ringnut 387 des dritten Konnektorteils 380 eingeschoben wird und anschließend die beiden Konnektorteile 380, 280 gegensinnig gegeneinander verdreht werden. Dabei kann der Stift 288 des zweiten Konnektorteils 280 in einer Nut 388 des dritten Konnektorteils 380 geführt sein. Das Einrasten des Bajonettverschlusses, insbesondere des Stifts 288 am Ende der Nut 188 in eine entsprechende Ausnehmung, ist vorzugsweise haptisch und/oder akustisch wahrnehmbar.

Das Set 10 kann wie folgt verwendet werden.

Zunächst kann die Verweilkanüle 200 über das Kanülenrohr 340 der Kanüle 300 geschoben und das zweite Konnektorteil 280 mit dem dritten Konnektorteil 380 verbunden werden. Das spitze distale Ende 340a des Kanülenrohrs 340 ragt dabei über das distale Ende 240a des Verweilkanülenrohrs 240 hervor. Diese beiden Komponenten können gemeinsam in einen Körper eingestochen werden. Dabei kann einerseits über den Zuspritzschlauch 360 eine Flüssigkeit in das Kanülenrohr 340 sowie über die seitliche Öffnung 349 in den Ringspalt 510 zwischen Kanülenrohr 340 und Innenwandung des Verweilkanülenrohrs 240 gespritzt werden, welche die Ultraschallsichtbarkeit des Kanülenrohrs 340 beim Einführen in den Körper verbessert, welche jedoch aufgrund der zweiten Dichtung 500 nicht in den Körper austreten kann. Andererseits kann über das Stimulationskabel 350 eine Stimulation erfolgen, indem Strompulse an das Kanülenrohr 340 angelegt werden, um Information über die Positionierung der Spitze des Kanülenrohrs 340 im Körper zu erhalten.

Ist die Kanüle 300 wie gewünscht positioniert, kann die Verbindung zwischen dem dritten Konnektorteil 380 und dem zweiten Konnektorteil 280 gelöst und die Kanüle 300 aus der Verweilkanüle 200 herausgezogen werden, während die Verweilkanüle 200 im Körper positioniert bleibt. Anschließend kann der Schlauch 140 mit dem Stimulationsdraht 120 der Vorrichtung 100 zur Nervenstimulation durch die Verweilkanüle 200 vorgeschoben werden, bis das distale Ende der Vorrichtung 100a, insbesondere die Stimulationselektrode 130 aus dem distalen Ende 240a der Verweilkanüle 240 austritt. Es kann nun sowohl eine Stimulation bei Anlegen entsprechender Strompulse über das Stimulationskabel 150 als auch ein Zuspritzen von Flüssigkeit, beispielsweise von Anästhetikum, über den Zuspritzschlauch 160 erfolgen. Die flexible Verweilkanüle 200 ermöglicht dabei eine langfristige Anordnung im Körper sowie eine Positionierung der Vorrichtung 100 auch in der Nähe von ansonsten über die Körperoberfläche schwer zugänglichen Nerven.

### Bezugszeichenliste

- 10: Set
- 100: Vorrichtung
- 100a: distales Ende
- 120: Stimulationsdraht
- 120a: distales Ende
- 120b: proximales Ende
- 122: Biegeabschnitt
- 123: zurückgeführter Abschnitt
- 125: Crimphülse
- 130: Stimulationselektrode
- 132: Außenfläche
- 134: Befestigungsabschnitt
- 136: axiale Durchgangsöffnung
- 140: Schlauch
- 140a: distales Ende
- 140b: proximales Ende
- 144: Markierung
- 146: Spalt
- 148: Abschnitt
- 149: seitliche Öffnung
- 150: Stimulationskabel
- 152: Ader
- 154: elektrischer Steckanschluss
- 160: Zuspritzschlauch
- 164: Spritzenanschluss
- 170: Gehäuse
- 172: Griffmulde
- 180: erstes Konnektorteil
- 180a: distales Ende
- 181: erste Kontaktfläche
- 182: distale Fläche
- 185: erster Vorsprung
- 187: Ringnut
- 187a: Bodenfläche
- 188: Nut
- 200: Verweilkanüle
- 240: Verweilkanülenrohr
- 240a: distales Ende
- 240b: proximales Ende
- 280: zweites Konnektorteil
- 280a: distales Ende
- 280b: proximales Ende
- 281: zweite Kontaktfläche
- 282: proximale Fläche
- 283: Wulst
- 285: zweiter Vorsprung
- 288: Stift
- 300: Kanüle
- 325: Crimphülse
- 340: Kanülenrohr
- 340a: distales Ende
- 340b: proximales Ende
- 349: seitliche Öffnung
- 350: Stimulationskabel
- 352: Ader
- 354: elektrischer Steckanschluss
- 360: Zuspritzschlauch
- 364: Spritzenanschluss
- 370: Gehäuse
- 380: drittes Konnektorteil
- 380a: distales Ende
- 381: dritte Kontaktfläche
- 382: distale Fläche
- 385: dritter Vorsprung
- 387: Ringnut
- 387a: Bodenfläche
- 388: Nut
- 400: erste Dichtung
- 410: Ringspalt
- 500: zweite Dichtung
- 510: Ringspalt
- AD: Durchmesser des Stimulationsdrahts
- AS: Außendurchmesser des Schlauchs
- IS: Innendurchmesser des Schlauchs
- AE: Außendurchmesser der Stimulationselektrode
- AV: Außendurchmesser des Verweilkanülenrohrs
- IV: Innendurchmesser des Verweilkanülenrohrs
- AK: Außendurchmesser des Kanülenrohrs
- IK: Innendurchmesser des Kanülenrohrs
- L: Länge

## Patentansprüche

1. Set (10) zur Nervenstimulation mit einer Vorrichtung (100) zur Nervenstimulation mit einem distalen Ende (100a), wobei die Vorrichtung (100) ein elektrisch leitendes Stimulationselement und einen Kanal zur Zufuhr eines Fluids umfasst, und mit einer Verweilkanüle (200) mit einem Verweilkanülenrohr (240) mit einem distalen Ende (240a) und einem proximalen Ende (240b), wobei die Vorrichtung (100) ein erstes Konnektorteil (180) und die Verweilkanüle (200) ein zu dem ersten Konnektorteil (180) komplementäres zweites Konnektorteil (280) aufweist, wobei durch Konnektieren des ersten Konnektorteils (180) und des zweiten Konnektorteils (280) die Vorrichtung (100) in einer axialen Position festlegbar ist, in welcher das distale Ende (100a) der Vorrichtung (100) über das distale Ende (240a) der Verweilkanüle (200) hervorragt, wobei im konnektierten Zustand des ersten Konnektorteils (180) und des zweiten Konnektorteils (280) das erste Konnektorteil (180) und das zweite Konnektorteil (280) derart miteinander verbunden sind, dass eine umlaufende erste Dichtung (500) gebildet ist,
**dadurch gekennzeichnet, dass** das zweite Konnektorteil (280) an einem distalen Ende (280a) das Verweilkanülenrohr (240) abdichtend umschließt und sich in Richtung auf ein proximales Ende (280b) aufweitet, so dass angrenzend an das proximale Ende (280b) des zweiten Konnektorteils (280) ein Ringspalt (410) zwischen dem zweiten Konnektorteil (280) und der eingeführten Vorrichtung (100) gebildet ist und dass das erste Konnektorteil (180) eine in Richtung auf ein distales Ende (180a) offene Ringnut (187) aufweist, in welche im konnektierten Zustand des ersten Konnektorteils (180) und des zweiten Konnektorteils (280) das proximale Ende (280b) des zweiten Konnektorteils (280) eintaucht.

2. Set nach Anspruch 1,
**dadurch gekennzeichnet, dass** die erste Dichtung (500) durch Anlage einer ersten Kontaktfläche (181) des ersten Konnektorteils (180) an einer zweiten Kontaktfläche (281) des zweiten Konnektorteils (280) gebildet ist.

3. Set nach Anspruch 2,
**dadurch gekennzeichnet, dass** das erste Konnektorteil (180) eine distale Fläche (182) aufweist, auf welcher ein in axialer Richtung vorstehender umlaufender erster Vorsprung (185) angeordnet ist, und das zweite Konnektorteil (280) eine proximale Fläche (182) aufweist, auf welcher ein in axialer Richtung vorstehender umlaufender zweiter Vorsprung (285) angeordnet ist, und die erste Kontaktfläche (181) auf der Außenseite des ersten Vorsprungs (185) und die zweite Kontaktfläche (281) auf der Innenseite des zweiten Vorsprungs (285) oder alternativ die erste Kontaktfläche (181) auf der Innenseite des ersten Vorsprungs (185) und die zweite Kontaktfläche (182) auf der Außenseite des zweiten Vorsprungs (285) angeordnet ist.

4. Set nach einem der Ansprüche 2 bis 3,
**dadurch gekennzeichnet, dass** die erste Kontaktfläche (181) einen in Richtung auf die zweite Kontaktfläche (281) vorstehenden umlaufenden Wulst und/oder die zweite Kontaktfläche (281) einen in Richtung auf die erste Kontaktfläche (181) vorstehenden umlaufenden Wulst (283) aufweist.

5. Set nach einem der vorhergehenden Ansprüche,
**dadurch gekennzeichnet, dass** der erste Vorsprung (185) auf einer Bodenfläche (187a) der Ringnut (187) angeordnet ist.

6. Set nach einem der vorhergehenden Ansprüche,
**dadurch gekennzeichnet, dass** der Kanal der Vorrichtung (100), insbesondere der Schlauch (140) der Vorrichtung (100), eine seitliche Öffnung (149) aufweist, welche insbesondere im konnektierten Zustand des ersten Konnektorteils (180) und des zweiten Konnektorteils (280) in den Ringspalt (410) mündet.

7. Set nach einem der vorhergehenden Ansprüche,
**dadurch gekennzeichnet, dass** das Konnektieren des ersten Konnektorteils (180) und des zweiten Konnektorteils (280) nach Art eines Bajonettverschlusses erfolgt, wobei vorzugsweise das Einrasten des Bajonettverschlusses haptisch und/oder akustisch wahrnehmbar ist.

8. Set nach einem der vorhergehenden Ansprüche,
**dadurch gekennzeichnet, dass** das Stimulationselement als elektrisch leitenden Stimulationsdraht (120) mit einem Durchmesser (AD), einem distalen Ende (120a) und einem proximalen Ende (120b), und der Kanal als ein elektrisch isolierender Schlauch (140) mit einem Innendurchmesser (IS), einem Außendurchmesser (AS), einem distalen Ende (140a) und einem proximalen Ende (140b) ausgebildet ist, wobei der Stimulationsdraht (120) in dem Schlauch (140) angeordnet ist, wobei der Innendurchmesser (IS) des Schlauchs (140) größer ist als der Durchmesser (AD) des Stimulationsdrahts (120), so dass ein Spalt (146) zur Zufuhr von Fluid in dem Schlauch (410) gebildet ist, wobei an dem distalen Ende (120a) des Stimulationsdrahts (120) eine Stimulationselektrode (130) elektrisch leitend angeordnet ist, welche über das distale (140a) Ende des Schlauchs (140) vorsteht.

9. Set nach Anspruch 8,
**dadurch gekennzeichnet, dass** das proximale Ende (140b) des Schlauchs (140) mit der Verbindungsstelle zu dem Stimulationskabel (150) und der Verbindungsstelle zu dem Zuspritzschlauch (160) in einem Gehäuse (170) angeordnet sind, wobei das Stimulationskabel (150) und der Zuspritzschlauch (160) proximal, insbesondere parallel zur Längsachse des Schlauchs (140), aus dem Gehäuse (170) herausgeführt sind, und das erste Konnektorteil (180) an dem Gehäuse (170) angeordnet ist, insbesondere einstückig mit diesem verbunden ist.

10. Set nach einem der vorhergehenden Ansprüche,
**dadurch gekennzeichnet, dass** das Set (10) weiterhin eine starre Kanüle (300) mit einem Kanülenrohr (340) mit einem distalen Ende (340a) und einem proximalen Ende (340b) zum Einstich in einen Körper umfasst, auf welche die Verweilkanüle (200) vor dem Einstich aufschiebbar ist und welche an ihrem proximalen Ende (340b) ein zu dem zweiten Konnektorteil (280) komplementäres drittes Konnektorteil (380) aufweist, wobei durch Konnektieren des dritten Konnektorteils (380) und des zweiten Konnektorteils (280) die Kanüle (300) in einer axialen Position festlegbar ist, in welcher das distale Ende (340a) des Kanülenrohrs (340) über das distale Ende (240a) der Verweilkanüle (200) hervorragt, wobei das dritte Konnektorteil (380) und das zweite Konnektorteil (280) derart miteinander verbunden sind, dass eine umlaufende zweite Dichtung (500) gebildet ist.

11. Set nach Anspruch 10,
**dadurch gekennzeichnet, dass** die zweite Dichtung (500) durch Anlage einer dritten Kontaktfläche (381) des dritten Konnektorteils (380) an der zweiten Kontaktfläche (281) des zweiten Konnektorteils (280) gebildet ist.

12. Set nach Anspruch 3 und Anspruch 11,
**dadurch gekennzeichnet, dass** das dritte Konnektorteil (380) eine distale Fläche (382) aufweist, auf welcher ein in axialer Richtung vorstehender umlaufender dritter Vorsprung (385) angeordnet ist, und die dritte Kontaktfläche (381) auf der Außenseite des dritten Vorsprungs (385) und die zweite Kontaktfläche (281) auf der Innenseite des zweiten Vorsprungs (285) oder alternativ die dritte Kontaktfläche (381) auf der Innenseite des dritten Vorsprungs (385) und die zweite Kontaktfläche (281) auf der Außenseite des zweiten Vorsprungs (285) angeordnet ist.

13. Set nach einem der Ansprüche 11 oder 12,
**dadurch gekennzeichnet, dass** die dritte Kontaktfläche (381) einen in Richtung auf die zweite Kontaktfläche (281) vorstehenden umlaufenden Wulst und/oder die zweite Kontaktfläche (281) einen in Richtung auf die dritte Kontaktfläche (381) vorstehenden umlaufenden Wulst (283) aufweist.

14. Set nach einem der Ansprüche 10 bis 13 und nach Anspruch 5,
**dadurch gekennzeichnet, dass** das dritte Konnektorteil (380) eine in Richtung auf ein distales Ende (380a) offene Ringnut (387) aufweist, in welche im konnektierten Zustand des dritten Konnektorteils (380) und des zweiten Konnektorteils (280) das proximale Ende (280b) des zweiten Konnektorteils (280) eintaucht, wobei vorzugsweise der dritte Vorsprung (385) auf einer Bodenfläche (387a) der Ringnut (387) angeordnet ist..

15. Set nach Anspruch 14,
**dadurch gekennzeichnet, dass** das Kanülenrohr (340) eine seitliche Öffnung (349) aufweist, welche insbesondere im konnektierten Zustand des dritten Konnektorteils (380) und des zweiten Konnektorteils (280) in den Ringspalt (510) mündet.

16. Set nach einem der Ansprüche 10 bis 15,
**dadurch gekennzeichnet, dass** das Konnektieren des dritten Konnektorteils (380) und des zweiten Konnektorteils (280) nach Art eines Bajonettverschlusses erfolgt, wobei vorzugsweise das Einrasten des Bajonettverschlusses haptisch und/oder akustisch wahrnehmbar ist.

17. Set nach einem der Ansprüche 10 bis 16,
**dadurch gekennzeichnet, dass** das Kanülenrohr (340) aus einem elektrisch leitenden Material gefertigt ist und ein elektrisch leitendes Stimulationskabel (350) auf der Außenseite des Kanülenrohrs (340) elektrisch leitend verbunden ist und wobei ein Zuspritzschlauch (360) abschnittsweise über das proximale Ende (340b) des Kanülenrohrs (340) abdichtend gestülpt ist.

18. Set nach Anspruch 17,
**dadurch gekennzeichnet, dass** das proximale Ende (340b) des Kanülenrohrs (340) mit der Verbindungsstelle zu dem Stimulationskabel (350) und der Verbindungsstelle zu dem Zuspritzschlauch (360) in einem Gehäuse (370) angeordnet sind, wobei das Stimulationskabel (350) und der Zuspritzschlauch (360) proximal, insbesondere parallel zur Längsachse des Kanülenrohrs (340), aus dem Gehäuse (370) herausgeführt sind, und das dritte Konnektorteil (380) an dem Gehäuse (370) angeordnet ist, insbesondere einstückig mit diesem verbunden ist.

## Claims

1. Set (10) for nerve stimulation comprising a device (100) for nerve stimulation with a distal end (100a), wherein the device (100) comprises an electrically conductive stimulation element and a channel for supplying a fluid, and comprising an indwelling cannula (200) with an indwelling cannula tube (240) having a distal end (240a) and a proximal end (240b), wherein the device (100) has a first connector part (180) and the indwelling cannula (200) has a second connector part (280) complementary to the first connector part (180), wherein, by connecting the first connector part (180) and the second connector part (280), the device (100) can be fixed in an axial position in which the distal end (100a) of the device (100) projects beyond the distal end (240a) of the indwelling cannula (200), wherein, in the connected state of the first connector part (180) and the second connector part (280), the first connector part (180) and the second connector part (280) are connected to one another in such a way that a circumferential first seal (500) is formed, **characterized in that** the second connector part (280) sealingly surrounds the indwelling cannula tube (240) at a distal end (280a) and widens in the direction toward a proximal end (280b), so that, adjacent to the proximal end (280b) of the second connector part (280), an annular gap (410) is formed between the second connector part (280) and the inserted device (100), and **in that** the first connector part has an annular groove (187) open in the direction toward a distal end (180a), into which, in the connected state of the first connector part (180) and the second connector part (280), the proximal end (280b) of the second connector part (280) dips.

2. Set according to claim 1,
**characterized in that** the first seal (500) is formed by abutment of a first contact surface (181) of the first connector part (180) against a second contact surface (281) of the second connector part (280).

3. Set according to claim 2,
**characterized in that** the first connector part (180) has a distal surface (182) on which a circumferential first projection (185) projecting in the axial direction is arranged, and the second connector part (280) has a proximal surface (182) on which a circumferential second projection (285) projecting in the axial direction is arranged, and the first contact surface (181) is arranged on the outside of the first projection (185) and the second contact surface (281) is arranged on the inside of the second projection (285), or alternatively the first contact surface (181) is arranged on the inside of the first projection (185) and the second contact surface (182) is arranged on the outside of the second projection (285).

4. Set according to one of claims 2 to 3,
**characterized in that** the first contact surface (181) has a circumferential bead projecting in the direction toward the second contact surface (281) and/or the second contact surface (281) has a circumferential bead (283) projecting in the direction toward the first contact surface (181).

5. Set according to one of the preceding claims,
**characterized in that** the first projection (185) is arranged on a bottom surface (187a) of the annular groove (187).

6. Set according to one of the preceding claims,
**characterized in that** the channel of the device (100), in particular the tube (140) of the device (100), has a lateral opening (149), which opens in particular into the annular gap (410) in the connected state of the first connector part (180) and the second connector part (280).

7. Set according to one of the preceding claims,
**characterized in that** the connecting of the first connector part (180) and the second connector part (280) is effected in the manner of a bayonet lock, wherein preferably the engagement of the bayonet lock is perceptible haptically and/or acoustically.

8. Set according to one of the preceding claims,
**characterized in that** the stimulation element is designed as an electrically conductive stimulation wire (120) having a diameter (AD), a distal end (120a), and a proximal end (120b), and the channel is designed as an electrically insulating tube (140) having an inner diameter (IS), an outer diameter (AS), a distal end (140a), and a proximal end (140b), wherein the stimulation wire (120) is arranged in the tube (140), wherein the inner diameter (IS) of the tube (140) is greater than the diameter (AD) of the stimulation wire (120), so that a gap (146) for supplying fluid is formed in the tube (410), wherein a stimulation electrode (130) is electrically conductively arranged at the distal end (120a) of the stimulation wire (120), said stimulation electrode projecting beyond the distal end (140a) of the tube (140).

9. Set according to claim 8,
**characterized in that** the proximal end (140b) of the tube (140), together with the connection point to the stimulation cable (150) and the connection point to the injection tube (160), is arranged in a housing (170), wherein the stimulation cable (150) and the injection tube (160) are led proximally out of the housing (170), in particular parallel to the longitudinal axis of the tube (140), and the first connector part (180) is arranged on the housing (170), in particular integrally connected thereto.

10. Set according to one of the preceding claims,
**characterized in that** the set (10) further comprises a rigid cannula (300) with a cannula tube (340) having a distal end (340a) and a proximal end (340b) for insertion into a body, onto which the indwelling cannula (200) can be pushed before insertion and which has, at its proximal end (340b), a third connector part (380) complementary to the second connector part (280), wherein, by connecting the third connector part (380) and the second connector part (280), the cannula (300) can be fixed in an axial position in which the distal end (340a) of the cannula tube (340) projects beyond the distal end (240a) of the indwelling cannula (200), wherein the third connector part (380) and the second connector part (280) are connected to one another in such a way that a circumferential second seal (500) is formed.

11. Set according to claim 10,
**characterized in that** the second seal (500) is formed by abutment of a third contact surface (381) of the third connector part (380) against the second contact surface (281) of the second connector part (280).

12. Set according to claim 3 and claim 11,
**characterized in that** the third connector part (380) has a distal surface (382) on which a circumferential third projection (385) projecting in the axial direction is arranged, and the third contact surface (381) is arranged on the outside of the third projection (385) and the second contact surface (281) is arranged on the inside of the second projection (285), or alternatively the third contact surface (381) is arranged on the inside of the third projection (385) and the second contact surface (281) is arranged on the outside of the second projection (285).

13. Set according to one of claims 11 or 12,
**characterized in that** the third contact surface (381) has a circumferential bead projecting in the direction toward the second contact surface (281) and/or the second contact surface (281) has a circumferential bead (283) projecting in the direction toward the third contact surface (381).

14. Set according to one of claims 10 to 13 and according to claim 5,
**characterized in that** the third connector part (380) has an annular groove (387) open in the direction toward a distal end (380a), into which, in the connected state of the third connector part (380) and the second connector part (280), the proximal end (280b) of the second connector part (280) dips, wherein preferably the third projection (385) is arranged on a bottom surface (387a) of the annular groove (387).

15. Set according to claim 14,
**characterized in that** the cannula tube (340) has a lateral opening (349), which opens in particular into the annular gap (510) in the connected state of the third connector part (380) and the second connector part (280).

16. Set according to one of claims 10 to 15,
**characterized in that** the connecting of the third connector part (380) and the second connector part (280) is effected in the manner of a bayonet lock, wherein preferably the engagement of the bayonet lock is perceptible haptically and/or acoustically.

17. Set according to one of claims 10 to 16,
**characterized in that** the cannula tube (340) is made of an electrically conductive material and an electrically conductive stimulation cable (350) is electrically conductively connected to the outside of the cannula tube (340), and wherein an injection tube (360) is slipped sealingly in sections over the proximal end (340b) of the cannula tube (340).

18. Set according to claim 17,
**characterized in that** the proximal end (340b) of the cannula tube (340), together with the connection point to the stimulation cable (350) and the connection point to the injection tube (360), is arranged in a housing (370), wherein the stimulation cable (350) and the injection tube (360) are led proximally out of the housing (370), in particular parallel to the longitudinal axis of the cannula tube (340), and the third connector part (380) is arranged on the housing (370), in particular integrally connected thereto.

## Revendications

1. Ensemble (10) de stimulation nerveuse comprenant un dispositif (100) de stimulation nerveuse ayant une extrémité (100a) distale, dans lequel le dispositif (100) comprend un élément de stimulation, conducteur de l'électricité, et un conduit d'apport d'un fluide, et comprenant une canule (200) de séjour ayant un tube (240) de canule de séjour, ayant une extrémité (240a) distale et une extrémité (240b) proximale, dans lequel le dispositif (100) a une première pièce (180) de connecteur et la canule (200) de séjour une deuxième pièce (280) de connecteur complémentaire de la première pièce (180) de connecteur, dans lequel, par connexion de la première pièce (180) de connecteur et de la deuxième pièce (280) de connecteur, le dispositif (100) peut être fixé dans une position axiale, dans laquelle l'extrémité (100a) distale du dispositif (100) est en saillie de l'extrémité (240a) distale de la canule (200) de séjour, dans lequel, dans l'état connecté de la première pièce (180) de connecteur et de la deuxième pièce (280) de connecteur, la première pièce (180) de connecteur et la deuxième pièce (280) de connecteur sont assemblées l'une à l'autre de manière à former une première étanchéité (500) faisant le tour,
**caractérisé en ce que** la deuxième pièce (280) de connecteur entoure avec étanchéité, à une extrémité (280a) distale, le tube (240) de canule de séjour et s'élargit dans la direction d'une extrémité (280b) proximale de manière à former, au voisinage de l'extrémité (280b) proximale de la deuxième pièce (280) de connecteur, un intervalle (410) annulaire entre la deuxième pièce (280) de connecteur et le dispositif (100) inséré et de manière à ce que la première pièce (180) de connecteur ait une rainure (187) annulaire, ouverte en direction d'une extrémité (180a) distale, dans laquelle, dans l'état connecté de la première pièce (180) de connecteur et de la deuxième pièce (280) de connecteur, l'extrémité (280b) proximale de la deuxième pièce (280) de connecteur pénètre.

2. Ensemble suivant la revendication 1,
**caractérisé en ce que** la première étanchéité (500) est formée par application d'une première surface (181) de contact de la première pièce (180) de connecteur sur une deuxième surface (281) de contact de la deuxième pièce (280) de connecteur.

3. Ensemble suivant la revendication 2,
**caractérisé en ce que** la première pièce (180) de connecteur a une surface (182) distale, sur laquelle est disposée une première saillie (185) faisant le tour, en saillie dans la direction axiale, et la deuxième pièce (280) de connecteur a une surface (182) proximale, sur laquelle est disposée une deuxième saillie (285) faisant le tour, en saillie dans la direction axiale, et la première surface (181) de contact est disposée sur la face extérieure de la première saillie (185) et la deuxième surface (281) de contact est disposée sur la face intérieure de la deuxième saillie (285) ou, en variante, la première surface (181) de contact est disposée sur la face intérieure de la première saillie (185) et la deuxième surface (182) de contact est disposée sur la face extérieure de la deuxième saillie (285).

4. Ensemble suivant l'une des revendications 2 à 3,
**caractérisé en ce que** la première surface (181) de contact a un bourrelet faisant le tour, en saillie dans la direction de la deuxième surface (281) de contact, et la deuxième surface (281) de contact a un bourrelet (283) faisant le tour, en saillie dans la direction de la première surface (181) de contact.

5. Ensemble suivant l'une des revendications précédentes,
**caractérisé en ce que** la première saillie (185) est disposée sur une surface (187a) de fond de la rainure (187) annulaire.

6. Ensemble suivant l'une des revendications précédentes,
**caractérisé en ce que** le conduit du dispositif (100), en particulier le conduit (140) souple du dispositif (100), a une ouverture (149) latérale, qui débouche, en particulier dans l'état connecté de la première pièce (180) de connecteur et de la deuxième pièce (280) de connecteur, dans l'intervalle (410) annulaire.

7. Ensemble suivant l'une des revendications précédentes,
**caractérisé en ce que** la connexion de la première pièce (180) de connecteur et de la deuxième pièce (280) de connecteur, s'effectue à la manière d'une fermeture à baïonnette, dans lequel, de préférence, l'enclenchement de la fermeture à baïonnette est perceptible haptiquement et/ou acoustiquement.

8. Ensemble suivant l'une des revendications précédentes,
**caractérisé en ce que** l'élément de stimulation est sous la forme d'un fil (120) de stimulation, conducteur de l'électricité, ayant un diamètre (AD), une extrémité (120a) distale et une extrémité (120b) proximale, et le conduit est sous la forme d'un tube (140) souple, isolant électriquement, ayant un diamètre (IS) intérieur, un diamètre (AS) extérieur, une extrémité (140a) distale et une extrémité (140b) proximale, dans lequel le fil (120) de stimulation est disposé dans le tube (140) souple, dans lequel le diamètre (IS) intérieur du tube (140) souple est plus grand que le diamètre (AD) du fil (120) de stimulation de manière à former un intervalle (146) d'apport de fluide dans le tube (140) souple, dans lequel, à l'extrémité (120a) distale du fil (120) de stimulation, est disposée une électrode (130) de stimulation, conductrice de l'électricité, qui est en saillie de l'extrémité (140a) distale du tube (140) souple.

9. Ensemble suivant la revendication 8,
**caractérisé en ce que** l'extrémité (140b) proximale du tube (140) souple, ayant le point de liaison au câble (150) de stimulation, et le point de liaison au tube (160) souple de pulvérisation, sont disposés dans un boîtier (170), dans lequel le câble (150) de stimulation et le tube (160) souple de pulvérisation sortent de manière proximale, en particulier parallèlement à l'axe longitudinal du tube (140) souple, du boîtier (170) et la première pièce (180) de connecteur est disposée sur le boîtier (170) en étant, en particulier, d'une pièce avec celui-ci.

10. Ensemble suivant l'une des revendications précédentes,
**caractérisé en ce que** l'ensemble (10) comprend en outre une canule (300) rigide ayant un tube (340) de canule, ayant une extrémité (340a) distale et une extrémité (340b) proximale pour la piqûre dans un corps, sur laquelle la canule (200) de séjour peut coulisser avant la piqûre et qui a, à son extrémité (340b) proximale, une troisième pièce (380) de connecteur, complémentaire de la deuxième pièce (280) de connecteur, dans lequel, par connexion de la troisième pièce (380) de connecteur et de la deuxième pièce (280) de connecteur, la canule (300) peut être fixée dans une position axiale, dans laquelle l'extrémité (340a) distale du tube (340) de canule est en saillie de l'extrémité (240a) distale de la canule (200) de séjour, dans lequel la troisième pièce (380) de connecteur et la deuxième pièce (280) de connecteur sont assemblées l'une à l'autre de manière à former une deuxième étanchéité (500) faisant le tour.

11. Ensemble suivant la revendication 10,
**caractérisé en ce que** la deuxième étanchéité (500) est formée par application d'une troisième surface (381) de contact de la troisième pièce (380) de connecteur à la deuxième surface (281) de contact de la deuxième pièce (280) de connecteur.

12. Ensemble suivant la revendication 3 et la revendication 11, **caractérisé en ce que** la troisième pièce (380) de connecteur a une surface (382) distale, sur laquelle est disposée une troisième saillie (385) faisant le tour, en saillie dans la direction axiale, et la troisième surface (381) de contact est disposée sur la face extérieure de la troisième saillie (385) et la deuxième surface (281) de contact sur la face intérieure de la deuxième saillie (285) ou, en variante, la troisième surface (381) de contact est disposée sur la face intérieure de la troisième saillie (385) et la deuxième surface (281) de contact est disposée sur la face extérieure de la deuxième saillie (285) de contact.

13. Ensemble suivant l'une des revendications 11 ou 12,
**caractérisé en ce que** la troisième surface (381) de contact a un bourrelet faisant le tour, en saillie dans la direction de la deuxième surface (281) de contact, et/ou la deuxième surface (281) de contact a un bourrelet (283) faisant le tour, en saillie dans la direction de la troisième surface (381) de contact.

14. Ensemble suivant l'une des revendications 10 à 13 et suivant la revendication 5,
**caractérisé en ce que** la troisième pièce (380) de connecteur a une rainure (387) annulaire, ouverte en direction d'une extrémité (380a) distale, dans laquelle, dans l'état connecté de la troisième pièce (380) de connecteur et de la deuxième pièce (280) de connecteur, l'extrémité (280b) proximale de la deuxième pièce (280) de connecteur pénètre, dans lequel, de préférence, la troisième saillie (385) est disposée sur une surface (387a) de fond de la rainure (387) annulaire.

15. Ensemble suivant la revendication 14,
**caractérisé en ce que** le tube (340) de canule a une ouverture (349) latérale qui, en particulier dans l'état connecté de la troisième pièce (380) de connecteur et de la deuxième pièce (280) de connecteur, débouche dans l'intervalle (510) annulaire.

16. Ensemble suivant l'une des revendications 10 à 15,
**caractérisé en ce que** la connexion de la troisième pièce (380) de connecteur et de la deuxième pièce (280) de connecteur s'effectue à la manière d'une fermeture à baïonnette, dans lequel, de préférence, l'enclenchement de la fermeture à baïonnette est perceptible haptiquement et/ou acoustiquement.

17. Ensemble suivant l'une des revendications 10 à 16,
**caractérisé en ce que** le tube (340) de canule est en un matériau conducteur de l'électricité et un câble (350) de stimulation conducteur de l'électricité est connecté, d'une manière conductrice de l'électricité, à la face extérieure du tube (340) de canule et dans lequel un tube (360) souple de pulvérisation est retroussé, de manière étanche, tronçon par tronçon, sur l'extrémité (340b) proximale du tube (340) de canule.

18. Ensemble suivant la revendication 17,
**caractérisé en ce que** l'extrémité (340b) proximale du tube (340) de canule, ayant le point de liaison au câble (350) de stimulation, et le point de liaison au tube (360) souple de pulvérisation, sont disposés dans un boîtier (370), dans lequel le câble (350) de stimulation et le tube (360) souple de pulvérisation sortent, de manière proximale, du boîtier (370), en particulier parallèlement à l'axe longitudinal du tube (340) de canule, et la troisième pièce (380) de connecteur est disposée sur le boîtier (370), en étant en particulier, d'une pièce avec celui-ci.
